# EUROPEAN PATENT APPLICATION

(11) **EP 2 732 886 A1**
(43) Date of publication of application: **21.05.2014**
(21) Application number: 12814730.3
(22) Date of filing: 06.06.2012
(51) Int. Cl.: B08B 3/08

(54) **CLEANING APPARATUS**

(30) Priority: 15.07.2011 JP 2011156442
(71) Applicant: Panasonic Corporation, Osaka 571-8501 (JP)
(72) Inventor: SANEMATSU, Wataru, Chuo-ku Osaka 540-6207 (JP)
(74) Representative: Grünecker, Kinkeldey, Stockmair & Schwanhäusser
(86) International application number: PCT/JP2012/064516
(87) International publication number: WO 2013/011761

(57) **Abstract**

A cleaning and purifying apparatus 40 cleans a cleaning object 51 in cleaning liquid generated by a plasma generator 1 and pours liquid from a water injection portion 60 toward the cleaning object 51 at the end of cleaning.

## Description

### TECHNICAL FIELD

The present invention relates to a cleaning apparatus that cleans a small appliance such as an electric shaver.

### BACKGROUND ART

One of conventionally known techniques performs discharge in a liquid containing bubbles to produce radicals and the like for reforming the liquid (see PTL 1, for example). In the discharge process in water disclosed in PTL 1, alternating pulse voltage is applied to electrodes which are opposed to each other and are not in contact with water in a discharge vessel, and an electrical field which is induced when the polarities are reversed is used to cause discharge in water. The discharge in water as described above generates effective components in bubbles to reform the liquid containing the bubbles.

### CITATION LIST

### PATENT LITERATURE

PATENT LITEARTURE 1: Japanese Patent Laid-open Publication No. 2001-9463

### SUMMARY OF INVENTION

However, the conventional techniques include the following problems.

Liquid to be processed contains various types of contamination, impurities, and the like. The electric resistance of the liquid greatly changes depending on these components in some cases. When the electric resistance of the liquid varies, the way that the discharge occurs varies even if a predetermined voltage is applied to between the electrodes. This prevents stable generation of plasma, thus resulting in variation in the amount of radicals produced. It is therefore hard to stably obtain a large amount of radicals.

Moreover, in the case of using the liquid reformed by generating radicals in such a manner as a cleaning liquid, the generated radicals enable biotreatment of water (wastewater) containing organic substances in the presence of activated sludge containing aerobic or anaerobic microorganisms and treatment of wastewater containing hardly-biodegradable substances. However, physical contamination existing in the liquid adheres to the object to be washed in some cases. Such a problem can be included also in conventional cleaning apparatuses which clean in a different way from plasma cleaning.

The present invention is made to solve the aforementioned problem, and an object of the present invention is to provide a cleaning apparatus which is capable of removing physical contamination adhering to an object to be cleaned.

A cleaning apparatus according to a first aspect of the present invention is a cleaning apparatus for cleaning a small electric device, including: a water injection portion pouring liquid toward a cleaning object at the end of cleaning after the cleaning object is cleaned in a cleaning liquid.

A cleaning apparatus according to a second aspect of the present invention is characterized in that the water injection portion pours the liquid when the cleaning object is exposed.

A cleaning apparatus according to a third aspect of the present invention is characterized in that the water injection portion is positioned above the liquid surface of the cleaning liquid.

A cleaning apparatus according to a fourth aspect of the present invention is characterized in that the water injection portion is positioned to surround the cleaning object.

A cleaning apparatus according to a fifth aspect of the present invention is characterized in that the cleaning apparatus includes a plasma generator and cleans the cleaning object in a cleaning liquid generated by the plasma generator, the plasma generator including: a liquid accommodation portion accommodating liquid including at least water; a gas accommodation portion accommodating gas; a partition wall which separates the liquid accommodation portion from the gas accommodation portion and includes a gas passage that allows the gas in the gas accommodation portion to pass through and introduces the gas to the liquid accommodation portion; a first electrode provided for the gas accommodation portion; a second electrode which is distant from the first electrode and at least a part of which on the side paired with the first electrode is in contact with the liquid in the liquid accommodation portion; a gas supply portion which supplies gas containing at least oxygen to the gas accommodation portion in a mode where the gas of the gas accommodation portion is pressure-fed to the liquid accommodation portion through the gas passage; and a plasma power supply supplying a predetermined voltage between the first and second electrodes and generating discharge between the first and second electrodes to turn into plasma, the gas introduced into the gas accommodation portion in the liquid accommodated in the liquid accommodation portion.

### BRIEF DESCRIPTION OF THE DRAWINGS

Fig. 1 is a partially cross-sectional view schematically illustrating the configuration of a plasma generator according to an embodiment of the present invention.
Fig. 2 is a diagram showing the relationship between potentials of first and second electrodes of the plasma generator according to the embodiment of the present invention.
Fig. 3 is a partial enlarged cross-sectional view schematically illustrating a state for explaining the operation of the plasma generator according to the embodiment of the present invention.
Fig. 4 is a partial enlarged cross-sectional view schematically illustrating the state subsequent to the state illustrated in Fig. 3.
Fig. 5 is a partially cross-sectional view schematically illustrating the configuration of a cleaning and purifying apparatus according to the embodiment of the present invention.
Fig. 6 is a perspective view illustrating a concrete example of the cleaning and purifying apparatus including the plasma generator according to the embodiment of the present invention.
Fig. 7 is a side cross-sectional view of the cleaning and purifying apparatus illustrated in Fig. 6.
Fig. 8 is a cross-sectional view taken along a line A-A of Fig. 7.
Fig. 9 is a side cross-sectional view of the cleaning and purifying apparatus including the plasma generator according to the embodiment of the present invention.

### MODES FOR CARRYING OUT THE INVENTION

Hereinbelow, a description is given of an embodiment of the present invention in detail with reference to the drawings.

A cleaning and purifying apparatus according to the embodiment is premised on the use of a plasma generator 1 capable of efficiently producing a large amount of radicals. Accordingly, the plasma generator 1 is described first.

### <Plasma Generator>

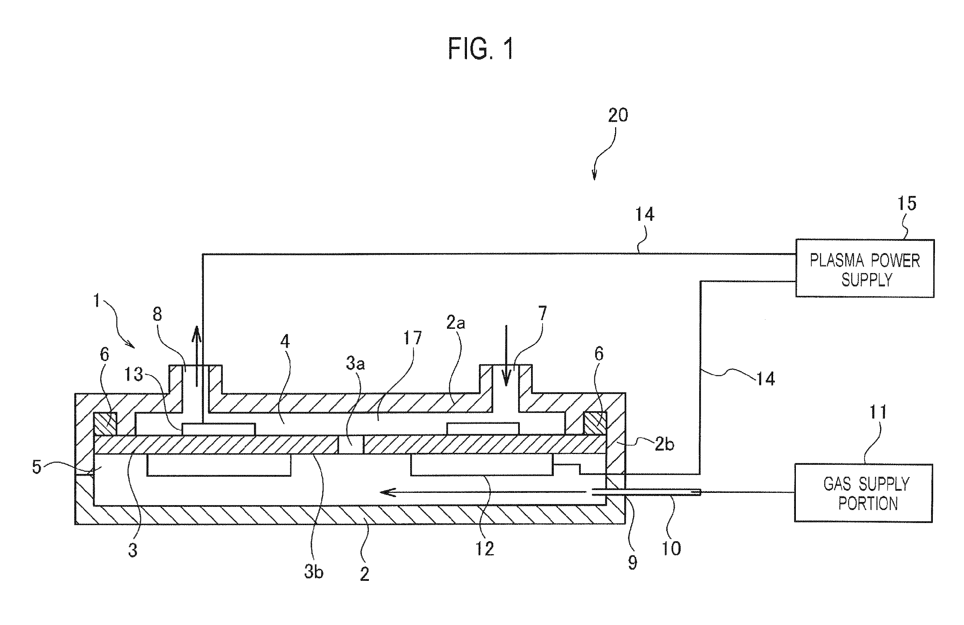

As illustrated in Fig. 1, the plasma generator 1 according to the embodiment includes a substantially cylindrical case member 2. The shape of the case member 2 is not limited to a cylindrical shape and may be a rectangular cylindrical shape. Within the case member 2, a ceramics member 3 is provided. The internal space of the case member 2 is partitioned by the ceramics member 3 into upper and lower regions. The region above the ceramic member 3 in the internal space of the case member 2 serves as a liquid accommodation portion 4 accommodating the liquid 17 including water. On the other hand, the region below the ceramics member 3 serves as a gas accommodation portion 5 accommodating gas. The ceramics member 3 corresponds to a partition wall portion separating the liquid and gas accommodation portions 4 and 5.

A ring-shaped seal material 6 is attached to the outer periphery of the liquid accommodation portion 4, which fills up gap between the case member 2 and ceramics member 3. This prevents the liquid 17 within the liquid accommodation portion 4 from leaking through the gap between the case and ceramics members 2 and 3 into the gas accommodation portion 5.

A top wall portion (the wall portion on the liquid accommodation portion 4 side) 2a of the case member 2 is provided with a liquid inlet port 7 through which the liquid 17 is introduced to the liquid accommodation portion 4. Moreover, the top wall portion 2a is provided with a liquid outlet port 8 through which the liquid 17 introduced into the liquid accommodation portion 4 is fed to the outside.

In lower part of a sidewall 2b of the case member 2, a gas inlet portion 9 is provided, which connects the gas accommodation portion 5 and the outside. A tube (gas inlet path) 10 is inserted in the gas inlet port 9. The gas accommodation portion 5 and a gas supply portion 11 are connected through the tube 10. In this embodiment, gas containing at least oxygen (O₂) is supplied from the gas supply portion 11 into the gas accommodation portion 5. The ceramics member 3 includes a gas passage 3a. Accordingly, the gas introduced from the gas supply portion 11 into the gas accommodation portion 5 and the like are fed through the gas passage 3a into the liquid accommodation portion 4.

As described above, the gas supply portion 11 has a function of supplying gas containing at least oxygen to the gas accommodation portion 5 in a mode where the gas in the gas accommodation portion 5 is pressure-fed through the gas passage 3a to the liquid accommodation portion 4. In this embodiment, the hole diameter of the gas passage 3a is set to about 1 to 10 µm so that the liquid 17 accommodated in the liquid accommodation portion 4 does not leak into the gas accommodation portion 5 through the gas passage 3a.

The plasma generator 1 includes a first electrode 12 and a second electrode 13. The first electrode 12 is provided for the gas accommodation portion 5. The second electrode 13 is provided away from the first electrode 12 so that at least a part thereof on the side paired with the first electrode 12 is in contact with the liquid 17 in the liquid accommodation portion 4. Specifically, the first and second electrodes 12 and 13 are doughnut-shaped and are provided for the gas and liquid accommodation portions 5 and 4, respectively.

As illustrated in Fig. 1, the doughnut-shaped first electrode 12 is located on a surface 3b of the ceramics member 3 on the gas accommodation portion 5 side with the center thereof set to the gas passage 3a. The surface of the first electrode 12 is coated with a dielectric material (not shown). The second electrode 13 is located in the liquid accommodation portion 4 so that at least a part thereof on the side paired with the first electrode 12 is in contact with the liquid 17 accommodated in the liquid accommodation portion 4. The second electrode 13 is also positioned with the center set to the gas passage 3a. In other words, the first and second electrodes 12 and 13 are arranged concentrically.

The doughnut-shaped first electrode 12 is provided for the gas accommodation portion 5 as described above, so that the first electrode 12 is not in contact with the liquid 17 introduced to the liquid accommodation portion 4. On the other hand, the doughnut-shaped second electrode 13 is provided for the liquid accommodation portion 4, so that the second electrode 13 (including at least a part of the side paired with the first electrode 12) is in contact with the liquid 17 introduced to the liquid accommodation portion 4.

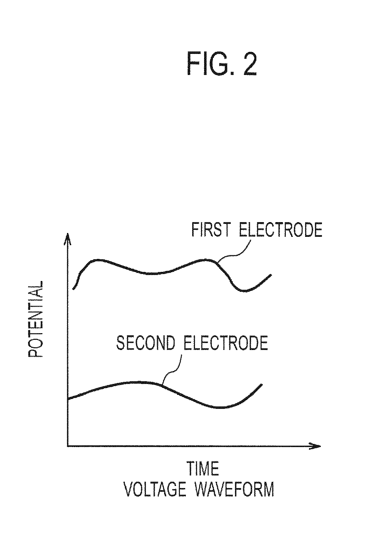

The first and second electrodes 12 and 13 are electrically connected to a plasma power supply 15 (see Fig. 1) through leads 14, and a predetermined voltage is applied to between the first and second electrodes 12 and 13. As illustrated in Fig. 2, the electrical potential of the second electrode 13 placed in the liquid 17 is set lower than that of the first electrode 12 placed in the gas to avoid the risk of electric shock.

Next, a description is given of the operation of the plasma generator 1 and a method of producing hydroxyl radicals.

In the mode where the gas in the gas accommodation portion 5 is pressure-fed to the liquid accommodation portion 4 through the gas passage 3a, first, the gas containing oxygen is supplied to the gas accommodation portion 5 (the step of supplying gas). As illustrated in Fig. 1, in the embodiment, air-based gas containing oxygen (the flow rate: about 0.01 to 1.0 L/min (10 to 1000 cc/min)) is fed to the gas accommodation portion 5 from the gas supply portion 11 through the tube 10. In this process, the pressure to feed the gas is set to about 0.0098 to 0.49 MPa (0.1 to 5 kgf/cm²).

The gas supply portion 11 has a function of supplying gas (air) in the atmosphere as described above. The flow rate of supplied gas is controlled by a flow rate controller provided for the gas supply portion 11. The gas supply portion 11 may have a function of supplying not only gas in the atmosphere but also another type of gas (for example, a gas having a different oxygen content) (a gas type controller). It is therefore possible to selectively supply one or plural types of gases among various types of gases.

When the gas is supplied to the gas accommodation portion 5, the pressure of the supplied gas is added to the atmospheric pressure, and the gas accommodation portion 5 then has a pressure of about 0.11 to 0.59 MPa (1.1 to 6 Kgf/cm²) into positive pressure. The positive pressure of the gas accommodation portion 5 forms a gas flow from the gas accommodation portion 5 through the gas passage 3a to the liquid accommodation portion 4. Moreover, the positive pressure of the gas accommodation portion 5 prevents the liquid 17 accommodated in the liquid accommodation portion 4 from leaking into the gas accommodation portion 5 through the gas passage 3a. When the gas containing oxygen is supplied as described above, a bubble 16 containing oxygen grows at an opening end 3c of the gas passage 3a on the liquid accommodation portion 4 side (the upper side in Fig. 1) (a step of growing a bubble).

Subsequently, the plasma power supply 15 applies a predetermined voltage between the first and second electrodes 12 and 13. The applied voltage is preferably a voltage enough to cause glow discharge at atmospheric pressure (power: about 10 to 100 W). When the predetermined voltage is applied to between the first and second electrodes 12 and 13, discharge occurs between the first and second electrodes 12 and 13 in a gas atmosphere having a pressure equal to or higher than atmospheric pressure. This discharge generates plasma in gas regions of the liquid 17 accommodated in the liquid accommodation portion 4 to generate ozone, hydroxyl radicals, and the like from water contained in the liquid and oxygen contained in the gas (a step of generating hydroxyl radicals). The technique to generate plasma at atmospheric pressure is reported in Document A (Sachiko Okazaki, "Atmospheric Pressure Glow Discharge Plasma and Its Application", REVIEW: 20th JSPF Annual Meeting).

In the embodiment, plasma is produced by generating a potential difference in gas within the bubbles 16 (gas in the vicinity of the gas-liquid boundary in the liquid 17 of the liquid accommodation portion 4). When a potential difference is generated in the vicinity of the gas-liquid boundary at which hydroxyl radicals are more likely to be produced (in the vicinity of the opening end 3c of the gas passage 3a exposed to the liquid 17), larger amounts of ozone, hydroxyl radicals, and the like are produced. Ozone, hydroxyl radicals, and the like can be produced not only in the bubbles 16 in the vicinity of the opening end 3c of the gas passage 3a exposed to the liquid 17 but also in the bubbles 16 fed into the liquid accommodation portion 4.

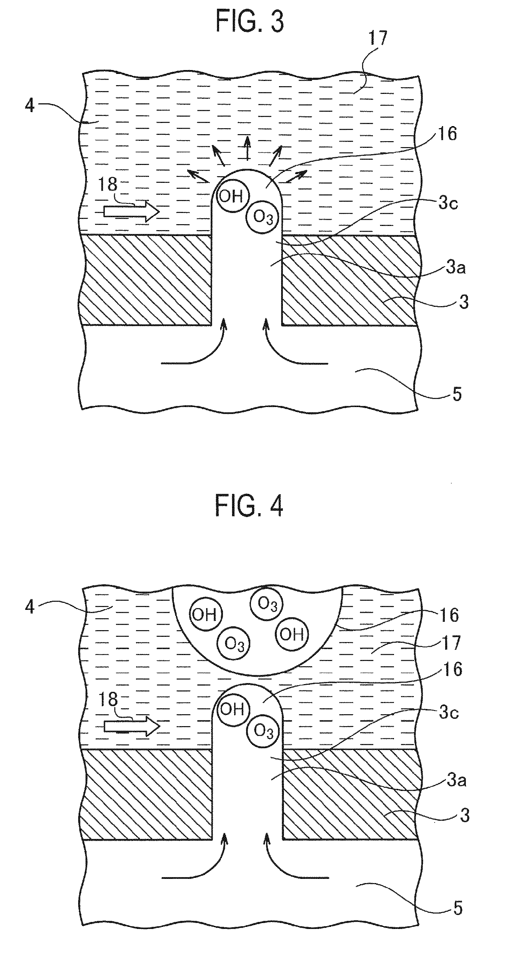

The thus generated ozone, hydroxyl radicals, and the like are fed to the liquid accommodation portion 4 along with the aforementioned flow of gas. The bubbles 16 containing hydroxyl radicals and the like are sheared off the ceramics member (partition wall) 3 by the flow of the liquid 17 in the liquid accommodation portion 4 to be released into the liquid 17 (a step of releasing bubbles). Specifically, the liquid accommodation portion 4 where the bubbles 16 grow includes a flow of the liquid 17 that is generated by introduction of the liquid 17 (see arrows 18 in Figs. 3 and 4). As illustrated in Fig. 4, when the liquid 17 flowing in the direction of the arrow 18 hits the growing bubble 16, the flow of the liquid 17 acts on the bubble 16 as a shearing force, so that the bubble 16 is released from the opening end 3c into the liquid 17. The bubbles 16 released into the liquid 17 are fine and are distributed all through the liquid 17 instead of being released directly into the atmosphere. Some of the distributed fine bubbles 16 are easily dissolved in the liquid 17. In this process, ozone and the like included in the bubbles 16 are dissolved into the liquid 17, increasing the ozone concentration of the liquid immediately.

According to Document B (Masayoshi Takahashi, Improvement in Water Environment by Micro-bubbles and Nano-babbles", Aquanet, 2004. 6), it is reported that many fine bubbles 16 containing ozone and various types of radicals are negatively charged. Accordingly, some other bubbles 16 are easily adsorbed onto organic substances, oil and fat substances, dyes, proteins, bacteria, and the like (not shown). The organic substances and the like in the liquid 17 are decomposed by ozone, various types of radicals, and the like which are dissolved in the liquid 17 or ozone, various types of radicals, and the like contained in the bubbles 16 which are adsorbed onto the organic substances and the like.

For example, hydroxyl radicals and the like have a comparatively large energy of about 120 kcal/mol. This energy is higher than the bond energy (100 kcal/mol at maximum) of a double bond between nitrogen atoms (N=N), a double bond between carbon atoms (C=C), a double bond between a carbon atom and a nitrogen atom (C=N), or the like. Accordingly, the bonds of the organic substances and the like composed of bonds of nitrogen, carbon, and the like are easily cut by the hydroxyl radicals and the like. The organic substances and the like are thus decomposed. Moreover, the ozone, hydroxyl radicals, and the like contributing to the decomposition of the organic substances and the like are not persistent unlike chlorine and disappear with time, which are environmentally-friendly substances.

In the plasma generator 1 according to the embodiment, the first electrode 12 is provided in the gas accommodation portion 5, and the second electrode 13 is provided so that at least a part of the second electrode 13 on the side paired with the first electrode 12 is in contact with the liquid in the liquid accommodation portion 4. By causing discharge between the first and second electrodes 12 and 13, plasma is generated in gas regions of the liquid 17 accommodated in the liquid accommodation portion 4, and hydroxyl radicals are generated from water contained in the liquid 17 and oxygen contained in the gas. According to the aforementioned configuration and method, it is possible to generate plasma between the first and second electrodes 12 and 13 with very little influence due to the electrical resistance of the liquid 17. Accordingly, it is possible to surely turn the gas into plasma and more stably produce a large amount of ozone, radicals, or the like.

According to the embodiment, the liquid 17 is introduced to the liquid accommodation portion 4, and the first electrode 12 generating plasma is provided for the gas accommodation portion 5 partitioned by the ceramics member 3. The first electrode 12 therefore does not come into contact with the liquid 17 at all and is therefore not influenced by the electrical resistance of the liquid 17. This allows the discharge between the first and second electrodes 12 and 13 to stably occur. Accordingly, the oxygen-contained gas introduced into the gas accommodation portion 5 is surely turned into plasma, thus stably producing ozone, hydroxyl radicals, and the like from water and oxygen.

Moreover, in the embodiment, ozone, hydroxyl radicals, and the like are produced in the gas within the bubbles 16 (gas in the vicinity of the gas-liquid boundary in the liquid 17 of the liquid accommodation portion 4). The gas containing ozone, hydroxyl radicals, or the like are diffused in the liquid 17 in the form of fine bubbles 16. Accordingly, ozone and various types of radicals can be efficiently fed into the liquid 17 in a very short period of time after being produced before the ozone and radicals disappear. When the fine bubbles 16 containing ozone and various types of radicals diffuse into the liquid 17, the ozone concentration of the liquid 17 is increased, and the bubbles 16 are adsorbed onto the organic substances and the like included in the liquid 17. It is therefore possible to efficiently decompose the organic substances, bacteria, and the like with ozone and the like dissolved in the liquid 17 or various radicals included in the adsorbed bubbles 16.

Moreover, use of the doughnut-shaped first and second electrodes 12 and 13 as the electrodes generating plasma can make compact the main body of the plasma generator 1 other than the plasma power supply 15 and gas supply portion 11. The plasma generator 1 can be therefore easily assembled to existing apparatuses. Moreover, in the case of mounting the plasma generator 1 on a new device, the space occupied by the plasma generator 1 can be minimized.

Moreover, when the gas supply portion 11 includes the gas type controller controlling the type of gas, the amounts of generated ozone and hydroxyl radicals and the like can be adjusted. In this process, if the gas supply portion 11 has a function of supplying air in the atmosphere, the gas can be easily supplied. Furthermore, when the flow-rate controller controls the flow rate of supplied gas, plasma can be stably generated.

### <Cleaning and Purifying Apparatus>

Next, a description is given of an example of cleaning and purifying apparatuses including the plasma generator 1.

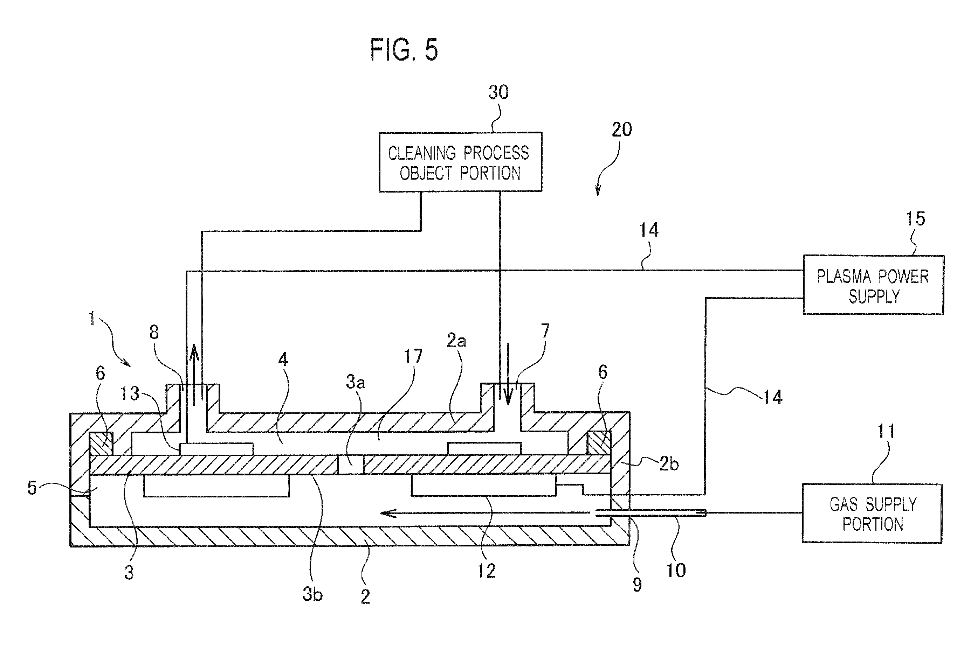

As illustrated in Fig. 5, a cleaning and purifying apparatus 20 includes the above-described plasma generator 1. The liquid inlet port 7 of the case member 2 accommodating the ceramics member 3 is connected to a tube (a liquid inlet path) 21 which introduces the liquid 17 having undergone the process from a cleaning process object portion (a cleaning object) 30. The liquid outlet port 8 is connected to a tube (a liquid outlet path) 22 which feeds the liquid in the liquid accommodation portion 4 to the cleaning process object portion 30.

Next, the operation of the above-described cleaning and purifying apparatus 20 is described.

As illustrated in Fig. 5, first, a predetermined flow rate of air-based gas containing oxygen is fed from the gas supply portion 11 into the gas accommodation portion 5 through the tube (the gas inlet path) 10. The gas accommodation portion 5 then is set to positive pressure to form a flow of gas from the gas accommodation portion 5 through the gas passage 3a to the liquid accommodation portion 4. In this process, the liquid 17 having undergone the process is introduced from the cleaning process object portion 30 through the tube (liquid inlet path) 21 and the liquid inlet port 7 into the liquid accommodation portion 4.

Subsequently, when the predetermined voltage is applied to between the first and second electrodes 12 and 13, discharge occurs between the first and second electrodes 12 and 13. The discharge generates plasma in gas regions of the liquid 17 in the liquid accommodation portion 4, and ozone, hydroxyl radicals, and the like are produced from water contained in the liquid 17 and oxygen contained in the gas (see Fig. 3). The generated ozone and various types of radicals are fed to the liquid accommodation portion 4 together with the aforementioned flow of gas. In this process, the growing bubbles are sheared off by the flow of the liquid 17 as described above to be released from the opening end 3c into the liquid as the fine bubbles 16.

The fine bubbles 16 released into the liquid distribute all through the liquid. In this process, some of the diffused fine bubbles 16 are easily dissolved into the liquid 17 together with ozone, hydroxyl radicals, and the like included in the bubbles 16, thus increasing the ozone concentration. Moreover, some of the bubbles 16 which include ozone, hydroxyl radicals, and the like are easily adsorbed onto organic substances and the like contained in the liquid 17. Furthermore, fine organic substances are adsorbed onto some of the bubbles 16.

The organic substances and the like in the liquid 17 are efficiently decomposed by ozone or radicals dissolved in the liquid 17, ozone or radicals included in the bubbles 16 adsorbed on the organic substances, and the like. The liquid 17 purified by decomposition of organic substances and the like is returned from the liquid outlet port 8 through the tube (liquid outlet path) 22 to the cleaning process object portion 30 to be used again.

In the example described above, the cleaning and purifying apparatus 20 is used in an application mode in which the liquid 17 is cleaned and purified within the case member 2 (application mode A). The cleaning and purifying apparatus 20 can be used in another application mode in which the liquid 17 including fine bubbles diffused is supplied to a predetermined device as a cleaning liquid (application mode B). In this case, the cleaning and purifying apparatus 20 operates in the following manner.

First, fine bubbles 16 including ozone, hydroxyl radicals, and the like are diffused in the liquid 17 introduced into the case member 2, and ozone, hydroxyl radicals, and the like included in the fine bubbles 16 are dissolved. In this process, fine organic substances are adsorbed onto some of the bubbles 16.

Subsequently, the liquid 17 is supplied to the cleaning process object portion 30 as the cleaning liquid. In the cleaning process object portion 30, the organic substances and the like are efficiently decomposed by ozone or radicals dissolved in the liquid 17, ozone or radicals included in the bubbles 16 adsorbed onto the organic substances, and the like.

In the case of using the cleaning and purifying apparatus in the application mode A, the cleaning and purifying apparatus is applicable to purification of various types of liquid such as hot water in a bath, rain water, sewage, and drainage. In the case of using the cleaning and purifying apparatus in the application mode B, the liquid 17 can be used as water used as the cleaning liquid in various household appliances such as washing machines and dishwashers, health home appliances such as mouth washing machines, sanitary appliances such as toilets. In addition to household appliances and the like, for example, the cleaning and purifying apparatus 20 is applicable to a wide range of industries including cleaning of foods, cleaning in the manufacturing process of industrial products, and the like.

The cleaning and purifying apparatus 20 includes the aforementioned plasma generator 1 as described above and thereby can efficiently generate a large amount of radicals. Moreover, when the cleaning and purifying apparatus 20 includes a position adjustment portion adjusting the position of the plasma generator 1, plasma can be further stabilized.

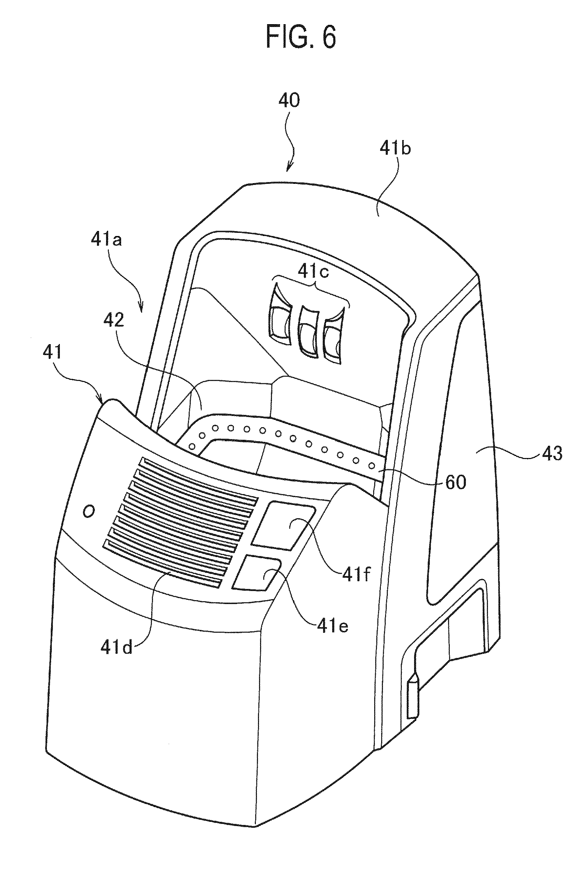

Next, with reference to Figs. 6 to 8, a description is given of a cleaning and purifying apparatus 40 including the plasma generator 1 in more detail. The cleaning and purifying apparatus 40 is a cleaning and purifying apparatus which is configured to clean a head portion 51 of an electric shaver 50 as a kind of hair removing devices and is used in the aforementioned application mode B. In this case, the head portion 51 of the electric shaver 50 corresponds to the cleaning process object portion 30.

As illustrated in Figs. 6 to 8, the cleaning and purifying apparatus 40 includes: a casing 41 including an opening 41a into which the electric shaver 50 is inserted with the head portion 51 down; and a receiver 42 receiving the head portion 51 inserted through the opening 41a. The cleaning and purifying apparatus 40 further includes: a tank 43 storing liquid; an overflow portion 44 communicating with the receiver 42; and a pump 45 circulating and supplying the liquid stored in the tank 43 to the liquid inlet port 7 of a water injection portion 60. The cleaning and purifying apparatus 40 further includes: a cartridge 46 having a filter 46a filtering liquid; an on-off valve 47 controlling the air sealing of the tank 43; and a circulation path through which the liquid circulates.

The circulation path includes the tube (liquid inlet path) 21, the tube (liquid outlet path) 22, a path 23 (a discharge path), a path 24, a path 25, and a path 26. The tube (liquid introduction path) 21 introduces the liquid stored in the tank 43 to the liquid inlet port 7. The tube (liquid inlet path) 22 introduces the liquid discharged from the liquid outlet port 8 to the receiver 42. The path 23 (discharge path) 23 introduces the liquid discharged from the receiver 42 to the cartridge 46. The path 24 introduces the liquid discharged from the overflow portion 44 to the cartridge 46. The path 25 introduces the liquid discharged from the cartridge 46 to the pump 45. The path 26 introduces the liquid fed from the pump 46 to the tank 43. The tank 43 is connected to the on-off valve 47 through the airtight path 27.

The casing 41 includes a stand portion 41b in the rear part. The stand portion 41b comes into contact with a grip portion 52 of the electric shaver 50. The casing 41 holds the electric shaver 50 inserted from the opening 41a in cooperation with the receiver 42. As illustrated in Fig. 6, a contact member 41c is provided on the front surface of the stand portion 41b. The contact member 41c is configured to detect that the electric shaver 50 is attached to the cleaning and purifying apparatus 40. The contact member 41c detects attachment of the electric shaver 50 based on a contact with a terminal 52a provided for the back of the grip portion 52. In addition to the detecting function, the contact member 41c has a function of outputting various types of control signals and driving power to the electric shaver 50.

Upper front part of the casing 41 accommodates a fan 48 that is used to dry the head portion 51 after cleaning. The front surface of the casing 41 is provided with a vent window 41d for the fan 48, an operation button 41e used to execute the cleaning operation, a lamp 41f displaying the operation state, and the like. The back side of the casing 41 serves as an attachment portion to which the tank 43 is attached and includes a joint ports 41g, 41h, and 41i, which are coupled with ports 43a, 43b, and 43c of the tank 43, respectively. The joint ports 41g, 41h, and 41i are connected to the tube (liquid introduction path) 21, the path 26, and the airtight path 27, respectively.

The receiver 42 has a concave profile along the shape of the head portion 51. The plasma generator 1 is provided in the back of the bottom wall portion of the receiver 42. The cleaning and purifying apparatus 40 may be provided with the position adjustment portion adjusting the position of the plasma generator 1. For example, the plasma generator 1 may be provided so as to be swung by an arm portion which is provided in back of the bottom wall portion and is adjusted by the position adjustment portion so as to be horizontally positioned. With such a configuration, the plasma generator 1 can be always positioned horizontally and therefore can generate plasma more stably.

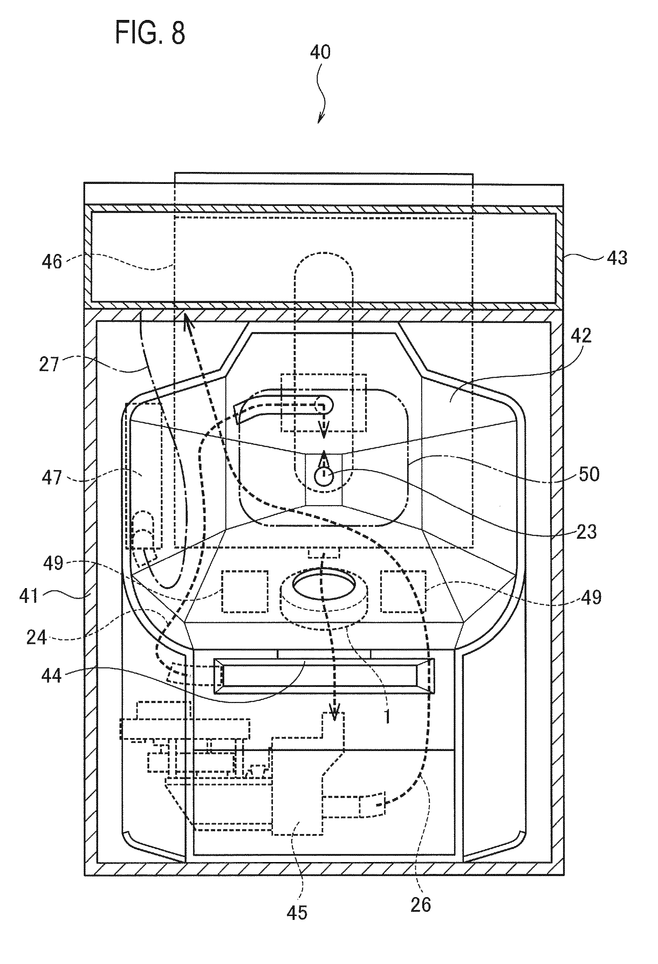

The plasma generator 1 includes: the liquid inlet port 7 connected to the tube (liquid inlet path) 21; and the liquid outlet port 8 connected to the tube (liquid outlet path) 22. In the bottom wall of the receiver 42, a supply port 41j connected to the tube (liquid outlet path) 22 and a discharge port 41k connected to the path 23 are provided. Moreover, a heater 49 is provided on the back side of the bottom wall portion of the receiver 42 (see Fig. 8). The heater 49 is configured to dry the head portion 51 in cooperation with the fan 48. In front of the receiver 42, the overflow portion 44 is provided. In this embodiment, the receiver 42 and overflow portion 44 are integrally formed. The inlet of the overflow portion 44 is connected to the receiver 42, and the outlet thereof is connected to the path 24. The path 24 extends from the outlet of the overflow portion 44 to the cartridge 46 through a relay port 42a provided in rear part of the receiver 42.

The tank 43 includes an outlet port 43a and an inlet port 43b and a ventilation port 43c in the front surface. The ventilation port 43c is configured to release the airtight condition and is opened and closed to control discharge of liquid from the outlet port 43a. The tank 43 is detachably provided on the back side of the casing 41. When the tank 43 is attached to the casing 41, the outlet port 43a is coupled with the joint portion 41g to be connected to the liquid inlet port 7 of the plasma generator 1 through the tube (liquid introduction path) 21. The inlet port 43b is coupled with the joint port 41h to be connected to a feeding port 45a of the pump 45 through the path 26. The ventilation port 43c is coupled with the joint port 41i to be connected to the on-off valve 47 through the airtight path 27.

The cartridge 46 is a substantially box-shaped member accommodating the filter 46a inside and is detachably provided in the lower back of the casing 41. The cartridge 46 includes an inlet port 46b in upper part and an outlet port 46c in front part. When the cartridge 46 is attached to the casing 41, the inlet port 46b is connected to the discharge port 41k through the path 23 (discharge path) and is connected to the outlet of the overflow portion 44 through the path 24. Moreover, the outlet port 46c is connected to an inlet port 45b of the pump 45 through the path 25.

With such a configuration, the fine bubbles 16 including ozone, hydroxyl radicals, and the like are diffused in the liquid introduced from the tank 43 to the plasma generator 1, thus producing a cleaning liquid. The produced cleaning liquid is supplied from the supply port 41j into the receiver 42 and is then supplied to the head portion 51 as the cleaning process object portion 30. It is therefore possible to efficiently decompose organic substances and the like adhering to the head portion 51 by ozone or radicals dissolved in the liquid (the cleaning liquid), ozone or radials included in the bubbles 16, and the like.

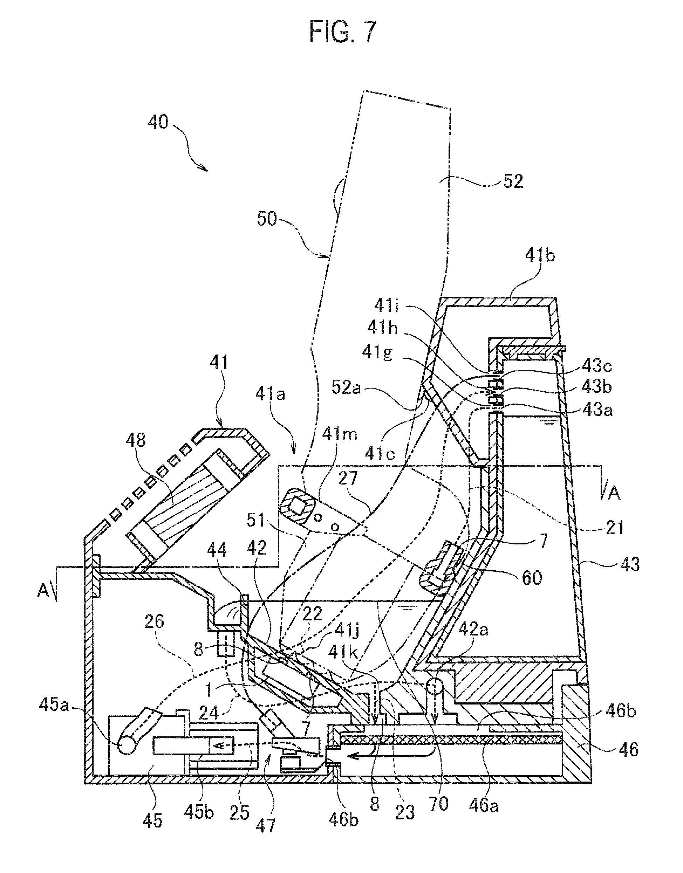

By the way, the cleaning liquid in the receiver 42 includes physical contamination such as hairs and sebum, and such physical contamination adheres to the head portion 51 in some cases. In the embodiment, therefore, in order to remove the physical contamination adhering to the head portion 51, the water injection portion 60 that pours liquid toward the head portion 51 is provided as illustrated in Figs. 6 and 7. The water injection portion 60 is a nozzle having a rectangular shape surrounding the head portion 51 on four sides. In the inner part of the water injection portion 60, plural orifices 41m are formed at predetermined intervals. An liquid inlet port 7 of the water injection portion 60 is connected to the tank 43 as described above.

The attachment position of the water injection portion 60 is not particularly limited, but the water injection portion 60 is preferably positioned above a liquid surface 70 of the cleaning liquid. The liquid surface 70 of the cleaning liquid herein refers to the liquid surface 70 that is assumed to be maximum when the liquid is stored in the receiver 42. When the water injection portion 60 is positioned above the liquid surface 70 of the liquid, the liquid can be poured without the resistance of the cleaning liquid. Moreover, as illustrated in Fig. 7, the head portion 51 is held by the casing 41 at a predetermined angle. Accordingly, it is preferable that the water injection portion 60 is attached at a predetermined angle. This allows the orifices 41m of the water injection portion 60 to directly face the surface of the head portion 51, so that the liquid can be evenly poured over the head portion 51.

Hereinafter, the operation of pouring water is described.

First, the head portion 51 is cleaned in the cleaning liquid within the receiver 42, and liquid is then poured from the water injection portion 60 toward the head portion 51 at the end of cleaning. Specifically, after the cleaning liquid within the receiver 42 is discharged from the liquid outlet port 8 to expose the head portion 51, liquid is introduced from the tank 43 to the water injection portion 60. The liquid therefore spouts from the orifices 41m of the water injection portion 60 toward the head portion 51, removing hairs and sebum adhering to the head portion 51. The liquid having spouted is discharged from the receiver 42 through the liquid outlet port 8 and is then introduced through the cartridge 46 to the tank 43.

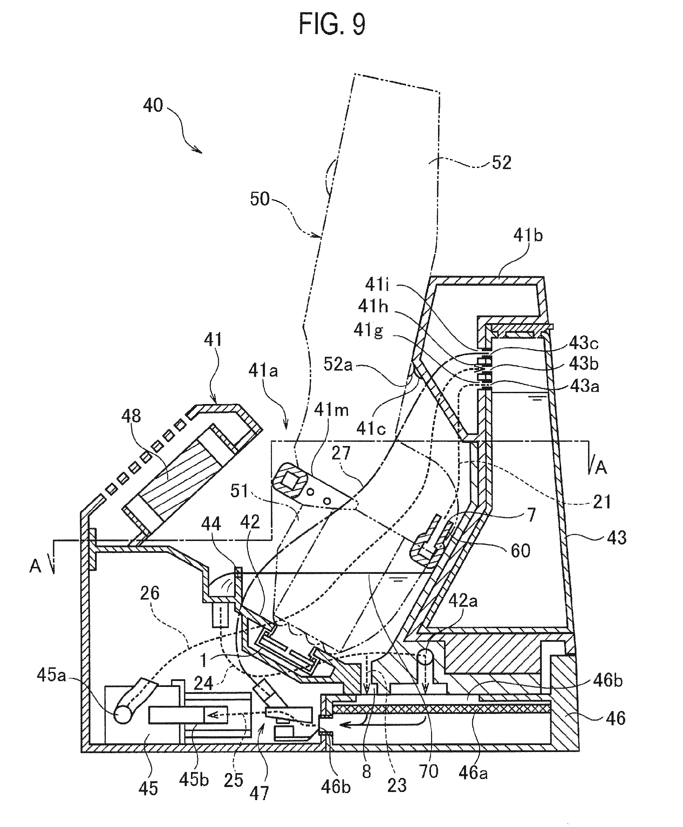

In the case of using the cleaning and purifying apparatus in the aforementioned application mode A, as illustrated in Fig. 9, the top wall of the casing member 2 of the plasma generator 1 may be opened, and the head portion 51 is immersed in the liquid 17 which is cleaned and purified in the casing member 2. In this configuration, organic substances and the like adhering to the head portion 51 are efficiently decomposed in a similar manner to the case of the application mode B. Moreover, physical contamination adhering to the head portion 51 can be also removed in a similar manner.

As described above, in the cleaning and purifying apparatus 40 according to the embodiment, the head portion 51 is cleaned in the cleaning liquid generated by the plasma generator 1, and liquid is poured from the water injection portion 60 toward the head portion 51 at the end of cleaning. It is therefore possible to efficiently produce a large amount of radicals and remove physical contamination adhering to the head portion 51.

Moreover, the water injection portion 60 is configured to pour liquid when the head portion 51 is exposed. This allows the head portion 51 to pour the liquid faster than the case where the head portion 51 is in the cleaning liquid, thus enhancing the cleaning performance.

Furthermore, the water injection portion 60 is positioned above the liquid surface 70 of the cleaning liquid. The liquid can be therefore poured without the resistance of the cleaning liquid, thus further enhancing the cleaning performance.

Still furthermore, the water injection portion 60 is positioned so as to surround the head portion 51. The liquid can be poured toward the head portion 51 on four sides with no dead zone. It is therefore possible to evenly remove physical contamination more effectively and shorten the cleaning period of time.

Hereinabove, the preferred embodiment of the present invention is described. However, the present invention is not limited to the aforementioned embodiment and can be variously changed. For example, in the example of the above-described embodiment, the partition wall including the gas passage is composed of the ceramics member. However, the material of the partition wall is not limited to ceramics. The partition wall may be a member composed of a proper material such as a glass plate separating gas from liquid and including a fine hole that has a diameter of about 1 to 10 µm and is formed by photoengraving and etching. The partition wall may include plural gas passages. Moreover, the specifications (shapes, sizes, layouts, and the like) of the liquid and gas accommodation portions and the other portions can be properly changed.

Certainly, the water injection portion 60 is not limited to the example shown above. Specifically, the water injection portion 60 only needs to pour liquid toward the head portion 51, and the profile, size, layout, and the like thereof can be changed. Moreover, it is preferable as described above that the injection of liquid is performed when the head portion 51 is exposed. However, the injection of liquid can be performed when the head portion 51 is in the cleaning liquid.

The above description is made based on the assumption that the cleaning and purifying generator 40 includes the plasma generator 1. However, the present invention is not limited to the configuration. Specifically, the present invention is also applicable to conventional cleaning apparatuses which do not perform plasma cleaning as long as the cleaning apparatuses are configured to clean small electric devices such as electric shavers.

The entire contents of Japanese Patent Application No. 2011-156442 (filed on: July 15, 2011) are incorporated herein by reference.

Hereinabove, the contents of the present invention are described with the embodiment. It is obvious to those skilled in the art that the present invention is not limited to the above description and can be variously changed and modified.

### INDUSTRIAL APPLICABILITY

The present invention is applicable to a cleaning apparatus which needs to remove physical contamination adhering to a cleaning process objet portion.

### EXPLANATION OF REFERENCE NUMERALS

- 1: PLASMA GENERATOR
- 3: CERAMICS MEMBER (PARTITION WALL)
- 3A: GAS PASSAGE
- 4: LIQUID ACCOMMODATION PORTION
- 5: GAS ACCOMMODATION PORTION
- 11: GAS SUPPLY PORTION
- 12: FIRST ELECTRODE
- 13: SECOND ELECTRODE
- 15: PLASMA POWER SUPPLY
- 20: CLEANING AND PURIFYING APPARATUS
- 30: CLEANING PROCESS OBJECT PORTION (CLEANING OBJECT)
- 40: CLEANING AND PURIFYING APPARATUS
- 60: WATER INJECTION PORTION
- 70: CLEANING LIQUID SURFACE

## Claims

1. A cleaning apparatus for cleaning a small electric device, comprising:
a water injection portion pouring liquid toward a cleaning object at the end of cleaning after the cleaning object is cleaned in a cleaning liquid.

2. The cleaning apparatus according to claim 1, wherein the water injection portion pours the liquid when the cleaning object is exposed.

3. The cleaning apparatus according to claim 1, wherein the water injection portion is positioned above the liquid surface of the cleaning liquid.

4. The cleaning apparatus according to claim 1, wherein the water injection portion is positioned to surround the cleaning object.

5. The cleaning apparatus according to any one of claims 1 to 4, wherein
the cleaning apparatus includes a plasma generator and cleans the cleaning object in a cleaning liquid generated by the plasma generator, the plasma generator including:
a liquid accommodation portion accommodating liquid including at least water;
a gas accommodation portion accommodating gas;
a partition wall which separates the liquid accommodation portion from the gas accommodation portion and includes a gas passage that allows the gas in the gas accommodation portion to pass through and introduces the gas to the liquid accommodation portion;
a first electrode provided for the gas accommodation portion;
a second electrode which is distant from the first electrode and at least a part of which on the side paired with the first electrode is in contact with the liquid in the liquid accommodation portion;
a gas supply portion which supplies gas containing at least oxygen to the gas accommodation portion in a mode where the gas of the gas accommodation portion is pressure-fed to the liquid accommodation portion through the gas passage; and
a plasma power supply supplying a predetermined voltage between the first and second electrodes and generating discharge between the first and second electrodes to turn into plasma, the gas introduced into the gas accommodation portion in the liquid accommodated in the liquid accommodation portion.
